# EUROPÄISCHE PATENTSCHRIFT

(11) **EP 0 805 649 B1**
(45) Veröffentlichungstag und Bekanntmachung des Hinweises auf die Patenterteilung: **03.09.2003**
(21) Anmeldenummer: 95936412.6
(22) Anmeldetag: 21.11.1995
(51) Int. Cl.: A61B 5/022

(54) **MANSCHETTE FÜR BLUTDRUCKMESSGERÄT**
SPHYGMOMANOMETER CUFF
MANCHETTE POUR APPAREIL DE MESURE DE LA TENSION

(43) Veröffentlichungstag der Anmeldung: 12.11.1997
(73) Patentinhaber: Disetronic Licensing AG, 3401 Burgdorf (CH)
(72) Erfinder: SCHAER, Andreas, CH-3323 Bäriswil BE (CH)
(74) Vertreter: Schwabe - Sandmair - Marx
(86) Internationale Anmeldenummer: CH9500274
(87) Internationale Veröffentlichungsnummer: WO97018750

(56) Entgegenhaltungen:
- EP-A- 0 009 789
- US-A- 3 765 405
- US-A- 5 243 991
- US-A- 5 396 894

## Beschreibung

Die vorliegende Erfindung bezieht sich auf eine Manschette für ein Blutdruckmessgerät, gemäss dem Oberbegriff des Anspruchs 1.

Manschetten für Blutdruckmessgeräte sind seit längerem bekannt. Sie bestehen aus einer flexiblen Hülle, einer Luftkammer, welche durch einen Schlauch mit einem Kontrollgerät verbindbar ist, und einer Haltevorrichtung. Die flexible Hülle wird durch ein weiches, meist waschbares Material gebildet, beinhaltet eine Luftkammer und ist Trägerin einer Haltevorrichtung. Die Luftkammer ist entweder in Form eines Beutels in der flexiblen Hülle untergebracht oder wird durch die flexible Hülle selbst gebildet. Die Haltevorrichtung dient dazu, die Manschette, wenn sie um den Arm, das Bein oder ein anderes Körperglied gewickelt wird, in die richtige Position zu bringen und zu halten. Die Haltevorrichtung selbst ist je nach Art der Manschette unterschiedlich ausgestaltet.

Bei Manschetten, welche vom Patienten selbst angebracht werden, ist am einen Ende der flexiblen Hülle eine Schleife, z.B. in Form eines Bügels, angeordnet, durch welche das andere Ende der Manschette gezogen wird. Der überlappende Teil der Manschette wird an der vom Patienten abgewandten Seite der flexiblen Hülle befestigt. Bekannt sind auch Schalenmanschetten, bei denen ein Teil der flexiblen Hülle aus einer flexiblen Halbschale besteht; mittels dieser flexiblen Halbschale wird eine lockere Halterung am betreffenden Körperglied ermöglicht, wobei zur endgültigen Fixierung zusätzlich ein Klettverschluss benötigt wird.

Manschetten, welche von einer Drittperson angebracht werden, bestehen meist nur aus einer flexiblen Hülle, welche um das betreffende Körperglied gewickelt werden und mittels eines Klettverschlusses oder einer anderen Haltevorrichtung um das Körperglied angebracht werden.

Allen diesen bekannten Manschetten ist gemeinsam, dass die flexible Hülle aus einer dem Patienten zugewandten inneren Seite und einer dem Patienten abgewandten äusseren Seite besteht. Ein Wechsel der Seiten ist nicht möglich. Der innere Teil der flexiblen Hülle kann nur an deren äusserem Teil befestigt werden. Sie sind daher entweder nur für ein linkes oder nur für ein rechtes Körperglied geeignet, weil der Schlauch, welcher Luftkammer und Kontrollgerät verbindet, oder die Luftkammer selbst, nicht für beide Körperglieder ideal positioniert werden können. Bei den einfacheren Ausführungen muss der Patient zudem den überlappenden Teil immer in gleicher Richtung durch die Schlaufe ziehen und an der äusseren Seite der flexiblen Hülle befestigen.

Die Blutdruckmessung sollte immer an demjenigen Glied erfolgen, bei welchem höhere Werte registriert werden. Dies ist für jeden Menschen unterschiedlich. Im weiteren muss besonders für diejenigen Patienten, welche ihren Blutdruck mehrmals während 24 Stunden messen, die Messung an demjenigen Glied vorgenommen werden, welches während des normalen Tagesablaufs am wenigsten belastet wird (in der Fachsprache wird dieses als das "nicht dominante Glied" bezeichnet), Auch das nicht dominante Glied ist individuell verschieden.

Eine Manschette, welche sowohl um ein linkes, wie auch um ein rechtes Körperglied gewickelt werden kann, ist aus der EP-A-0 251 273 bekannt. Nachteilig an dieser bekannten Manschette ist, dass sie zwei nicht veränderbare Seiten, eine dem Körper zugewandte und eine diesem abgewandte, voraussetzt. Da immer die gleiche Seite dem Körper zugewandt ist, muss beim Wechseln der Manschette vom linken zum rechten Körperglied der aufblasbare Beutel gedreht werden, damit der Schlauch am Blutdruckmessgerät in idealer Weise verläuft.

Eine weitere Manschette, welche sowohl um ein linkes, wie auch um ein rechtes Körperglied gewickelt werden kann, ist aus der EP-A-0 274 735 bekannt. Es handelt sich dabei um eine Manschette für die ambulante Blutdruckmessung, welche vom Patienten selbst angebracht wird. Bei dieser Ausführungsart wird der überlappende Teil, je nachdem, ob es sich um ein linkes oder rechtes Körperglied handelt, aus verschiedenen Richtungen durch die Schlaufe gezogen. Der aufblasbare Beutel, Welcher im inneren der flexiblen Hülle austauschbar gelagert ist, muss gedreht werden, damit der Schlauch zum Blutdruckmessgerät in idealer Weise verläuft.

Aus der US-A-5 243 991 sowie aus der US-A-5 396 894 ist jeweils eine Manschette für ein Blutdruckmessgerät bekannt, die eine flexible, im ausgerollten Zustand eine annähernd rechteckige Fläche aufweisende Hülle mit gegenüber liegenden kurzen Seiten umfasst. Die Hülle enthält in ihrem Inneren eine Luftkammer, die einen Anschluss zu einem Kontrollgerät aufweist. Auf ihren beiden Oberflächen sind Haltemittel vorgesehen zur Fixierung der Manschette auf einem Körperglied.

Eine Manschette für ein Blutdruckmessgerät ist schließlich aus der EP-A-0 009 789 bekannt, die ebenfalls eine flexible, im ausgerollten Zustand eine annähernd rechteckige Fläche aufweisende Hülle mit gegenüber liegenden kurzen Seiten umfasst. Die Hülle enthält in ihrem Inneren eine Luftkammer, die einen Anschluss zu einem Kontrollgerät aufweist. Auf ihren beiden Oberflächen sind magnetische oder klettenartige Haltemittel vorgesehen zur Fixierung der Manschette auf einem Körperglied, wobei ein Ende der Hülle unter Bildung einer Schlinge durch eine Schnalle geführt ist.

Hier will die Erfindung Abhilfe verschaffen. Der Erfindung liegt die Aufgabe zugrunde, eine Manschette zu schaffen, welche durch möglichst wenig Handgriffe für ein linkes oder rechtes Körperglied gebraucht werden kann, ohne dass die Wahl des Körpergliedes, an welchem die Manschette angebracht wird, die ideale Positionierung der Luftkammer oder des Schlauches beeinflusst und gleichzeitig die Befestigung der Manschette je nach Körperglied aus verschiedenen Richtungen möglich ist.

Die Erfindung löst die gestellte Aufgabe mit einer Manschette, welche die kennzeichnenden Merkmale des Anspruchs 1 aufweist.

Die durch die Erfindung erreichten Vorteile sind im wesentlichen darin zu sehen, dass dank der erfindungsgemässen Manschette durch wenige Handgriffe die gleiche Manschette sowohl für ein linkes wie auch für ein rechtes Körperglied verwendet werden kann.

Einige Ausführungsbeispiele der Erfindung sind in den Figuren dargestellt.

Es zeigen:
Fig. 1 eine Aufsicht auf eine Manschette nach dem Stand der Technik;
Fig. 2 eine Aufsicht auf eine erfindungsgemässe Manschette;
Fig. 3 eine Aufsicht auf eine bevorzugte Ausführungsform einer erfindungsgemässe Manschette;
Fig. 4 eine Seitenansicht der Manschette nach Fig. 3;
Fig. 5 eine Aufsicht einer weiteren Ausführungsform der erfindungsgemässen Manschette im zerlegten Zustand; und
Fig. 6 eine Aufsicht der Manschette nach Fig. 5 im zusammengesetzten Zustand.

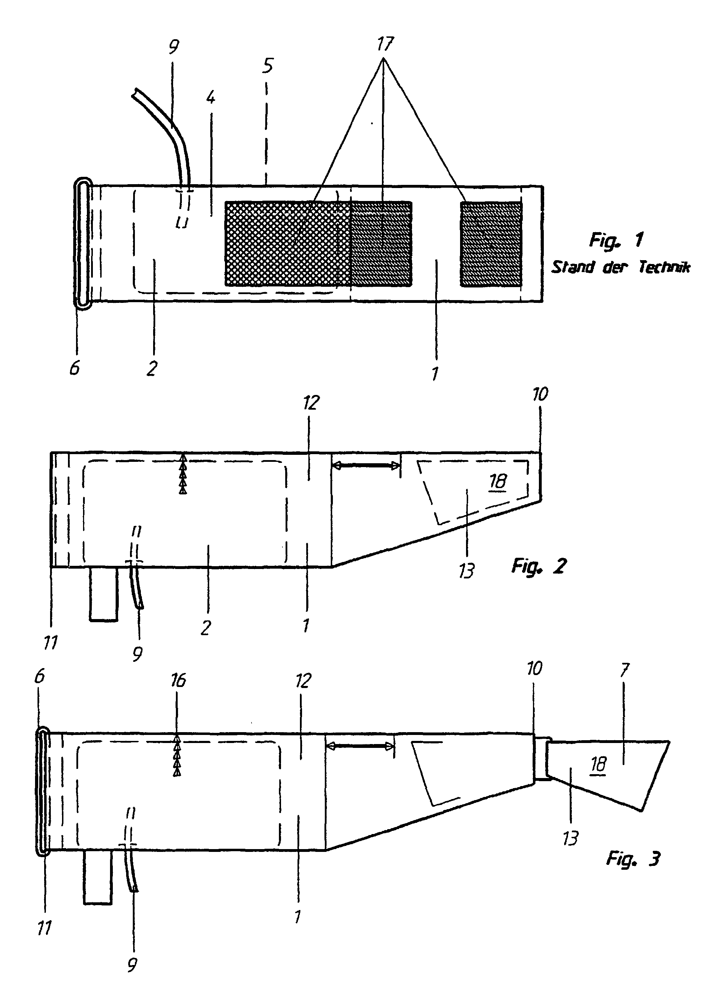

Wie in Fig. 1 dargestellt, besteht eine Manschette nach dem Stand der Technik aus einer flexiblen Hülle 1, einer Luftkammer 2, einem Schlauch 9 zu einem Kontrollgerät, wobei die flexible Hülle 1 eine dem Patienten zugewandte innere Seite 4 und eine dem Patienten abgewandte äussere Seite 5 beinhaltet, und einer Haltevorrichtung 6,17, welche aus einer metallischen Halteschlaufe 6 und einem auf der flexiblen Hülle 1 aufgebrachten Klett- oder Haken-Verschluss 17 besteht. Ein solcher Klettverschluss besteht aus einem Klettband und einem Flauschteil.

Die Einstellung der Länge und damit die Positionierung der Manschette am jeweiligen Glied erfolgt mit oder ohne Halteschlaufe 6 in Verbindung mit dem Klett- oder Haken-Verschluss 17.

Die in Fig. 2 gezeigte erfindungsgemässe Manschette für ein Blutdruckmessgerät besteht aus einer flexiblen, im ausgerollten Zustand eine annähernd rechteckige Fläche aufweisenden Hülle 1 mit einer ersten kurzen Seite 11 und einer zweiten kurzen Seite 10. Die Hülle 1 enthält in ihrem Innern eine Luftkammer 2, die einen Anschluss 9 zu einem Kontrollgerät aufweist, und verfügt auf ihren beiden Oberflächen 4,5 über Haltemittel 12 für die Fixierung der Manschette um ein Körperglied.
In der, der zweiten kurzen Seite 10 der Hülle 1 benachbarten trapezoiden Zone 18 sind auf beiden Oberflächen 4,5 Befestigungsmittel 13 zur temporären Befestigung an den Haltemitteln 12 vorgesehen. Da die Befestigungsmittel 13 in der Zone 18 auf beiden Oberflächen 4,5 (d.h. auf und hinter der Zeichenebene in Fig. 2) vorhanden sind werden vorzugsweise solche Befestigungsmittel gewählt, welche mit den üblicherweise vom Patienten getragenen Kleidungsstücken nicht (Flauschflächen) kooperieren können. Als Befestigungsmittel 13 kommen somit in erster Linie zusammenwirkende Noppen, Rillen, Magnetteile, Klebestreifen und ähnliche Elemente in Frage.

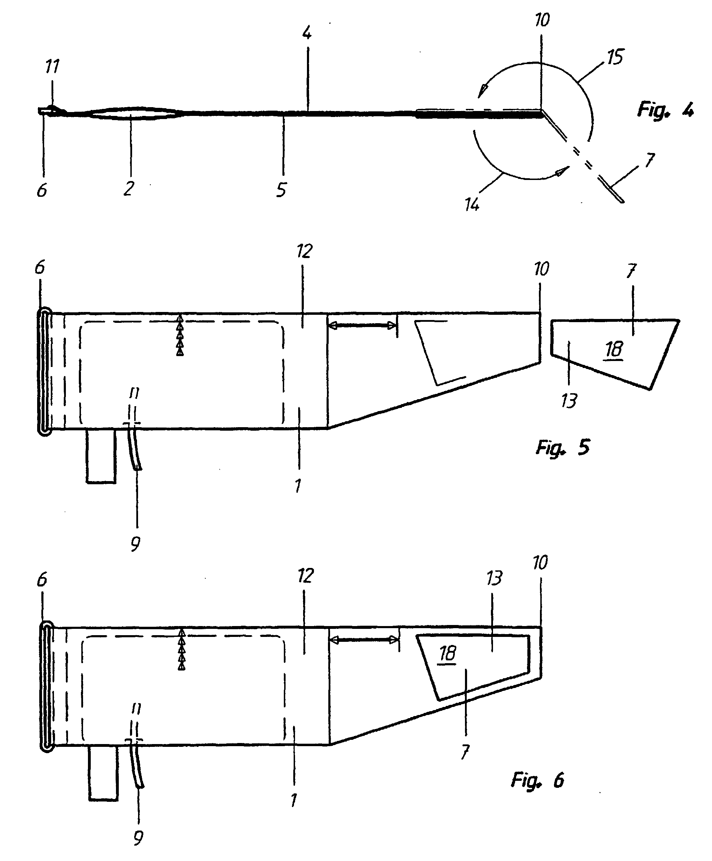

Bei der in Fig. 2 gezeigten Ausführungsform (ohne Halteschlaufe wie in Fig. 1) wird die flexible Hülle 1 um das gewünschte Körperglied gewickelt und anschliessend mittels der in Zone 18 vorhandenen Befestigungsmittel 13 an den Haltemitteln 12 der Hülle 1 befestigt.
Ist an der ersten kurzen Seite 11 - wie bei der Ausführung nach dem Stand der Technik gemäss Fig. 1 - eine Halteschlaufe 6 vorgesehen, wird die flexible Hülle 1 solange durch die Halteschlaufe 6 gezogen, bis die Innenseite (Oberfläche 4 in Fig. 4) der Hülle 1 das Körperglied umschliesst. Der durch die Halteschlaufe gezogene Teil der flexiblen Hülle 1 wird in der Gegenrichtung überschlagen und mittels der Befestigungsmittel 13 in Zone 18 auf der Aussenseite (Oberfläche 5 in Fig. 4) der flexiblen Hülle 1 befestigt.

Eine bevorzugte in den Fig. 3 und 4 dargestellte Ausführungsform der Erfindung unterscheidet sich von der in Fig. 2 gezeigten Ausführungsform dadurch, dass sie an der ersten kurzen Seite 11 eine metallischen Halteschlaufe 6 aufweist und an der zweiten kurzen Seite 10 einen auf der Innen- oder Aussenseite der flexiblen Hülle 1 befestigbaren viereckigen Lappen 7 als Zone 18 aufweist. Der Lappen 7 ist beidseitig mit Befestigungsmitteln 13, in Form von Kletten, versehen. Der Lappen 7 kann - dank seiner beidseitigen Beschichtung mit Kletten oder anderen analog wirkenden Strukturen - sowohl auf der dem Patienten zugewandten inneren Seite (Oberfläche 4), wie auch auf der dem Patienten abgewandten äusseren Seite (Oberfläche 5) der flexiblen Hülle 1 befestigt werden. Letztere Unterscheidung wird damit im Grunde hinfällig.

Bei einer weiteren - in den Fig. 5 und 6 dargestellten - Ausführungsform der Erfindung ist die Zone 18 als von der Hülle 1 getrennter Lappen 7 realisiert. Der separate Lappen 7 kann entweder auf der einen oder anderen Oberfläche (auf oder unter der Zeichenebene in Fig. 5) der Hülle 1 mittels seiner Befestigungsmittel 13 darauf befestigt werden. In Fig. 6 ist der trapezförmig ausgebildete Lappen auf der Oberseite der Hülle 1 - angrenzend an die zweite kurze Seite 10 - befestigt, so dass die Manschette nun gleich wie bei der Ausführungsform nach den Fig. 3 und 4 verwendet werden kann.

Im folgenden wird die Anwendung und Funktionsweise der erfindungsgemässen Manschette nach den Fig. 3 und 4 im Detail beschrieben.
Um die Manschette am betreffenden Körperglied zu positionieren, wird zuerst der Lappen 7 auf einer der beiden Oberflächen 4,5 der flexiblen Hülle 1 befestigt. Diese Befestigung hat nahe der zweiten kurzen Seite 10 der flexiblen Hülle 1 zu erfolgen. Der Lappen 7 besteht aus zwei Seiten, welche beide mit Befestigungsmitteln 13 ausgestattet sind. Die Befestigungsmittel 13 wirken mit den auf beiden Oberflächen 4,5 der Hülle 1 angebrachten Haltemitteln 12 zusammen. Als Kombinationen für die Haltemittel 12 / Befestigungsmittel 13 kommen bei dieser Ausführungsform Klettverschlüsse, Adhäsionsverschlüsse, magnetische Verschlüsse u.ä. in Frage.

In einer bevorzugten Ausführungsform ist der Lappen 7 selbst beidseitig mit Klettband versehen und die flexible Hülle 1 ist vollständig mit Flausch bedeckt, welcher mit dem Klettband zusammenwirkt. Wird der Lappen 7 auf der flexiblen Hülle 1 befestigt, verbleibt ihm eine freie, mit Klettband ausgestattete Seite. Diese Seite soll, wie unten gezeigt wird, dazu dienen, die flexible Hülle 1 richtig zu positionieren. Ist der Lappen 7 einmal auf der flexiblen Hülle 1 befestigt, erfolgt die Positionierung der erfindungsgemässen Manschette wie bei den bekannten Manschetten.

In der bevorzugten, in den Fig. 3 und 4 dargestellten Ausführungsform, befindet sich eine metallische Halteschlaufe 6 an der, dem Lappen 7 gegenüberliegenden kurzen Seite 11 der flexiblen Hülle 1. In dieser Ausführungsform wird diejenige Oberfläche 4,5 der Hülle 1, auf welcher der Lappen 7 befestigt wurde, zwingend zur Aussenseite der flexiblen Hülle 1. In Fig. 4 ist dies beispielsweise die eine Oberfläche 5. Die flexible Hülle 1 wird solange durch die Halteschlaufe 6 gezogen, bis die Innenseite (Oberfläche 4 in Fig. 4) der Hülle 1 das Körperglied umschliesst. Diverse Markierungen 16 auf der Aussenseite der flexiblen Hülle 1 erlauben es dem Anwender die Manschette so zu positionieren, dass die Mitte der Luftkammer 2 direkt über der zu messenden Arterie (z.B. der "Arteria brachialis") liegt. Ist die Luftkammer 2 richtig positioniert, wird der durch die Halteschlaufe 6 gezogene, den Lappen 7 tragende Teil der flexiblen Hülle 1 in der Gegenrichtung überschlagen und mittels der noch freien Seite des Lappens 7 an der Aussenseite der flexiblen Hülle 1 befestigt.

In der bevorzugten Ausführungsform gemäss den Fig. 3 und 4 ist die flexible Hülle 1 vollständig mit Flausch überdeckt, so dass der Lappen 7, welcher mit Klettband überdeckt ist, an jeder Stelle der flexiblen Hülle 1 befestigt werden kann. Denkbar ist auch, dass der Lappen 7 nur an bestimmten Stellen der flexiblen Hülle 1 befestigt werden kann, wie das bei den bekannten Manschetten üblich ist. Es ist von Vorteil, wenn der Lappen 7 an jeder Stelle der flexiblen Hülle 1 befestigt werden kann, weil andernfalls verschiedene Materialien benötigt werden und die Anordnung der verschiedenen Materialien genau berechnet werden muss, was höhere Herstellungskosten zur Folge hat.

Soll nun die Manschette um ein entgegengesetztes (z.B. rechter statt linker Oberarm) Körperglied befestigt werden, muss zuerst der Lappen 7 und der ihn tragende Teil von der Aussenseite der Hülle 1 gelöst und anschliessend durch die Halteschlaufe 6 gezogen werden. Daraufhin wird der Lappen 7 von der einen Oberfläche (5 in Fig. 4) in Richtung der Pfeile 14,15 zur anderen Oberfläche (4 in Fig. 4) der flexiblen Hülle 1 umgeklappt, so dass die ursprüngliche Innenseite zur Aussenseite wird. Die weitere Vorgehensweise stimmt mit der oben beschriebenen überein.

Verwendbar sind auch Manschetten ohne Halteschlaufen 6. Auch bei diesen Manschetten wird je nach Körperglied der Lappen 7 auf der einen oder anderen Oberfläche 4,5 der flexiblen Hülle 1 befestigt. Fehlt es allerdings an einer Halteschlaufe 6, wird der Lappen 7 an der jeweiligen Innenseite der flexiblen Hülle 1 befestigt, weil das Überschlagen in der Gegenrichtung entfällt.

## Patentansprüche

1. Manschette für ein Blutdruckmessgerät mit einer flexiblen, im ausgerollten Zustand eine annähernd rechteckige Fläche aufweisenden Hülle (1) mit einer ersten kurzen Seite (11) und einer zweiten kurzen Seite (10), wobei die Hülle (1) in ihrem Innern eine Luftkammer (2) enthält, die einen Anschluss (9) zu einem Kontrollgerät aufweist, und auf ihren beiden Oberflächen (4,5) Haltemittel (12) für die Fixierung der Manschette um ein Körperglied besitzt, wobei in der, der zweiten kurzen Seite (10) der Hülle (1) benachbarten Zone (18) auf beiden Oberflächen (4,5) Befestigungsmittel (13) zur temporären Befestigung an den Haltemitteln (12) vorgesehen sind,
**dadurch gekennzeichnet, dass**
die Zone (18) als ein an der zweiten kurzen Seite (10) der Hülle (1) befestigter Lappen (7) realisiert ist, der entweder zur einen Oberfläche (5) oder zur anderen Oberfläche (4) der Manschette klappbar und durch seine Befestigungsmittel (13) darauf befestigbar ist.

2. Manschette für ein Blutdruckmessgerät mit einer flexiblen, im ausgerollten Zustand eine annähernd rechteckige Fläche aufweisenden Hülle (1) mit einer ersten kurzen Seite (11) und einer zweiten kurzen Seite (10), wobei die Hülle (1) in ihrem Innern eine Luftkammer (2) enthält, die einen Anschluss (9) zu einem Kontrollgerät aufweist, und auf ihren beiden Oberflächen (4,5) Haltemittel (12) für die Fixierung der Manschette um ein Körperglied besitzt, wobei in der, der zweiten kurzen Seite (10) der Hülle (1) benachbarten Zone (18) auf beiden Oberflächen (4,5) Befestigungsmittel (13) zur temporären Befestigung an den Haltemitteln (12) vorgesehen sind,
**dadurch gekennzeichnet, dass**
die Zone (18) als von der Hülle (1) getrennter Lappen (7) realisiert ist, der entweder auf der einen Oberfläche (5) oder auf der anderen Oberfläche (4) der Manschette mittels seiner Befestigungsmittel (13) darauf befestigbar ist.

3. Manschette nach Anspruch 1 oder 2, **dadurch gekennzeichnet, dass** die Haltemittel (12) Haken und die Befestigungsmittel (13) Schlaufen sind, welche zusammen als Klettverschluss wirken.

4. Manschette nach Anspruch 1 oder 2, **dadurch gekennzeichnet, dass** die Haltemittel (12) Schlaufen und die Befestigungsmittel (13) Haken sind, welche zusammen als Klettverschluss wirken.

5. Manschette nach Anspruch 1 oder 2, **dadurch gekennzeichnet, dass** die Haltemittel (12) und die Befestigungsmittel (13) magnetische Elemente umfassen.

6. Manschette nach Anspruch 1 oder 2, **dadurch gekennzeichnet, dass** die Haltemittel (12) und die Befestigungsmittel (13) ein zusammenwirkender Adhäsivverschluss sind.

7. Manschette nach einem der Ansprüche 1 bis 6, **dadurch gekennzeichnet, dass** an der ersten kurzen Seite (11) der Hülle (1) eine Halteschlaufe (6) zur Einführung der zweiten kurzen Seite (10) vorgesehen ist.

8. Manschette nach einem der Ansprüche 1 bis 7, **dadurch gekennzeichnet, dass** die Zone (18) trapezoid ausgebildet ist.

9. Manschette nach einem der Ansprüche 1 bis 8, **dadurch gekennzeichnet, dass** die Haltemittel (12) und die Befestigungsmittel (13) flächenhaft kooperieren.

## Claims

1. Cuff for a sphygmomanometer [blood pressure measuring device] with a flexible casing (I) which has an approximately rectangular surface when unrolled, with a first short side (11) and a second short side (10), wherein the casing (1) contains in its interior an air chamber (2) which has a connection (9) to a monitoring device, and on its two surfaces (4, 5) has holding means (12) for fixing the cuff around a limb, wherein provided in the zone (18) adjacent to second short side (10) of the casing (1), on both surfaces (4, 5) there are fixing means (13) for temporary fastening to the holding means (12),
**characterised in that**
the zone (18) is realised as a tab (7) which is fastened to the second short side (10) of the casing (1) and which can be folded either onto one surface (5) or the other surface (4) of the cuff, and can be fastened to it by means of its fixing means (13).

2. Cuff for a sphygmomanometer with a flexible casing (1) which has an approximately rectangular surface when unrolled, with a first short side (11) and a second short side (10), wherein the casing (1) contains in its interior an air chamber (2) which has a connection (9) to a monitoring device, and on its two surfaces (4, 5) has holding means (12) for fixing the cuff around a limb, wherein provided in the zone (18) adjacent to second short side (10) of the casing (1), on both surfaces (4, 5) there are fixing means (13) for temporary fastening to the holding means (12),
**characterised in that**
the zone (18) is realised as a tab (7) which is separated from the casing (1), and which can be fastened onto either on one surface (5) or the other surface (4) of the cuff, by means of its fixing means (13) on it.

3. Cuff in accordance with claim 1 or 2, **characterised in that** the holding means (12) are hooks and the fixing means (13) are loops, which together work as a Velcro-type fastening.

4. Cuff in accordance with claim 1 or 2, **characterised in that** the holding means (12) are loops and the fixing means (13) are hooks, which together work as a Velcro-type fastening.

5. Cuff in accordance with claim 1 or 2, **characterised in that** the holding means (12) and the fixing means (13) comprise magnetic elements.

6. Cuff in accordance with claim 1 or 2, **characterised in that** the holding means (12) and the fixing means (13) are an interacting adhesive fastening.

7. Cuff in accordance with one of the claims 1 to 6, **characterised in that** provided on the first short side (11) of the casing (1) is a holding loop (6) for inserting the second short side (10).

8. Cuff in accordance with one of the claims 1 to 7, **characterised in that** the zone (18) is designed in a trapezoid form.

9. Cuff in accordance with one of the claims 1 to 8, **characterised in that** the holding means (12) and the fixing means (13) co-operate over their surfaces.

## Revendications

1. Manchette pour un appareil de mesure de la tension sanguine, comportant une enveloppe flexible (1) présentant dans l'état déroulé une surface approximativement rectangulaire avec un premier petit côté (11) et avec un deuxième petit côté (10), l'enveloppe (1) présentant dans son volume intérieur une chambre à air (2) qui comprend un raccord (9) vers un appareil de contrôle, et possédant sur ses deux surfaces (4, 5) des moyens de retenue (12) pour la fixation de la manchette autour d'un organe corporel, des moyens de fixation (13) pour la fixation temporaire sur les moyens de retenue (12) étant prévus dans la zone (18) voisine du deuxième petit côté (10) de l'enveloppe sur les deux surfaces (4, 5),
**caractérisée en ce que**
la zone (18) est réalisée sous la forme d'une patte (7) fixée sur le deuxième petit côté (10) de l'enveloppe (1), patte qui est rabattable soit vers l'une des surfaces (5) soit vers l'autre surface (4) de la manchette et susceptible d'y être fixée par ses moyens de fixation (13).

2. Manchette pour un appareil de mesure de la tension sanguine, comportant une enveloppe flexible (1) présentant dans l'état déroulé une surface approximativement rectangulaire avec un premier petit côté (11) et avec un deuxième petit côté (10), l'enveloppe (1) présentant dans son volume intérieur une chambre à air (2) qui comprend un raccord (9) vers un appareil de contrôle, et possédant sur ses deux surfaces (4, 5) des moyens de retenue (12) pour la fixation de la manchette autour d'un organe corporel, des moyens de fixation (13) pour la fixation temporaire sur les moyens de retenue (12) étant prévus dans la zone (18) voisine du deuxième petit côté (10) de l'enveloppe sur les deux surfaces (4, 5),
**caractérisée en ce que**
la zone (18) est réalisée sous la forme d'une patte (7) séparée de l'enveloppe (1) et susceptible d'être fixée soit sur l'une des surfaces (5) soit sur l'autre surface (4) de la manchette par ses moyens de fixation (13).

3. Manchette selon l'une ou l'autre des revendications 1 et 2, **caractérisée en ce que** les moyens de retenue (12) sont des crochets et les moyens de fixation (13) sont des bouclettes qui coopèrent à la manière d'une fermeture auto-agrippante.

4. Manchette selon l'une ou l'autre des revendications 1 et 2, **caractérisée en ce que** les moyens de retenue (12) sont des bouclettes et les moyens de fixation (13) sont des crochets qui coopèrent à la manière d'une fermeture auto-agrippante.

5. Manchette selon l'une ou l'autre des revendications 1 et 2, **caractérisée en ce que** les moyens de retenue (12) et les moyens de fixation (13) comprennent des éléments magnétiques.

6. Manchette selon l'une ou l'autre des revendications 1 et 2, **caractérisée en ce que** les moyens de retenue (12) et les moyens de fixation (13) forment une fermeture adhésive en coopération.

7. Manchette selon l'une des revendications 1 à 6, **caractérisée en ce qu'**une boucle de retenue (6) est prévue sur le premier petit côté (11) de l'enveloppe (1) pour introduire le deuxième petit côté (10).

8. Manchette selon l'une des revendications 1 à 7, **caractérisée en ce que** la zone (18) est réalisée sous forme trapézoïdale.

9. Manchette selon l'une des revendications 1 à 8, **caractérisée en ce que** les moyens de retenue (12) et les moyens de fixation (13) coopèrent de par leurs surfaces.
